# EUROPÄISCHE PATENTANMELDUNG

(11) **EP 4 023 952 A1**
(43) Veröffentlichungstag der Anmeldung: **06.07.2022**
(21) Anmeldenummer: 21217455.1
(22) Anmeldetag: 23.12.2021
(51) Int. Cl.: F24F 8/10, F24F 8/108, F24F 8/175, F24F 8/22, F24F 8/50, F24F 8/80, F24F 11/30, F24F 6/00, F24F 6/04, F24F 110/10, F24F 110/20, F24F 110/40, F24F 110/64, F24F 110/66, F24F 110/70, F24F 110/72, F24F 110/76

(54) **VORRICHTUNG ZUR VERBESSERUNG EINER RAUMLUFTQUALITÄT**

(30) Priorität: 29.12.2020 AT 511552020
(71) Anmelder: Schletterer Consult GmbH, 6235 Reith im Alpbachtal (AT)
(72) Erfinder: SCHLETTERER, Heinz, 6235 Reith im Alpbachtal (AT)
(74) Vertreter: Torggler & Hofmann Patentanwälte - Innsbruck

(57) **Zusammenfassung**

Vorrichtung (1) zur Verbesserung einer Raumluftqualität umfassend eine Fördereinrichtung (2), insbesondere einen drehzahlgeregelten Ventilator, zum Ein- und/oder Ausbringen von Luft in die und/oder aus der Vorrichtung (1), eine Filtereinrichtung (3) zum Filtern der in die Vorrichtung eingebrachten Luft, und eine Beduftungseinrichtung (4) zur Beduftung der eingebrachten Luft durch Holz, insbesondere durch Zirbenholz, und/oder ätherische Essenzen, vorzugweise eine von der Beduftungseinrichtung (4) gesonderte Bepflanzungseinrichtung (5) aufweist, welche zumindest eine lebende Pflanze (5a), vorzugsweise ein Moos, eine Flechte und/oder eine Blütenpflanze, umfasst.

## Beschreibung

Die Erfindung betrifft eine Vorrichtung zur Verbesserung einer Raumluftqualität.

Die Raumluftqualität eines Raumes hat einen starken Einfluss auf das Wohlbefinden und die Gesundheit von im Raum befindlichen Personen. Es ist demnach erstrebenswert, eine bestmögliche Raumluftqualität sicherzustellen, insbesondere da die meisten Menschen einen Großteil ihrer Lebenszeit in Innenräumen verbringen.

Die Raumluftqualität eines Raumes festzustellen ist nicht trivial, da die Raumluftqualität von unterschiedlichen Personen unterschiedlich empfunden wird. Als Ausgangspunkt zur Bestimmung der Raumluftqualität können allerdings verschiedenen Kennwerte einer Luft im Raum, beispielsweise Schadstoffbelastung oder Lufttemperatur, herangezogen werden. Insbesondere kann zumindest einer der folgenden Kennwerte zur Bestimmung der Raumluftqualität betrachtet werden:
- Luftfeuchtigkeit,
- Lufttemperatur,
- Luftdruck,
- CO₂-Gehalt,
- CO-Gehalt,
- Sauerstoffgehalt der Luft,
- Flüchtige organische Verbindungen (VOC), und/oder
- Feinstaubbelastung.

Ein Großteil der aus dem Stand der Technik bekannten Vorrichtungen zur Verbesserung einer Raumluftqualität haben den Nachteil, dass nur ein Teilaspekt der Raumluftqualität verbessert wird.

So tragen beispielsweise Raumerfrischer zur von einer Person subjektiv empfundenen Raumluftqualität bei, verbessern aber beispielsweise nicht die Schadstoffbelastung der Luft.

Vorrichtungen wie kontrollierte Wohnraumlüftungen können die Raumluftqualität zwar hinsichtlich mehrerer Aspekte verbessern, allerdings sind kontrollierte Wohnraumlüftungen sehr kostenintensiv, aufwendig zu installieren und nur schwer nachzurüsten.

Die Aufgabe der vorliegenden Erfindung besteht darin, eine gegenüber dem Stand der Technik verbesserte Vorrichtung zur Verbesserung einer Raumluftqualität anzugeben, welche die Nachteile des Stands der Technik zumindest teilweise vermeidet.

Diese Aufgabe wird gelöst durch die Merkmale des unabhängigen Anspruchs 1.

Erfindungsgemäß ist es demnach vorgesehen, dass eine Vorrichtung zur Verbesserung einer Raumluftqualität Folgendes umfasst:
- eine Fördereinrichtung zum Ein- und/oder Ausbringen von Luft in die und/oder aus der Vorrichtung,
- eine Filtereinrichtung zum Filtern der in die Vorrichtung eingebrachten Luft, und
- eine Beduftungseinrichtung zur Beduftung der eingebrachten Luft durch Holz, insbesondere durch Zirbenholz, und/oder ätherische Essenzen,
- vorzugweise eine von der Beduftungseinrichtung (4) gesonderte Bepflanzungseinrichtung aufweist, welche zumindest eine lebende Pflanze, vorzugsweise ein Moos, eine Flechte und/oder eine Blütenpflanze, umfasst.

Bevorzugt kann weiters eine Befeuchtungseinrichtung vorgesehen sein, die über Sensoren gesteuert die Luft optimal (insbesondere auf einen voreinstellbaren Sollwert) befeuchtet.

Dadurch ist es einerseits möglich, Schadstoffe wie Viren, Bakterien, Pollen, Umweltgifte oder Feinstaub aus der Luft zu filtern und andererseits die Luft zu beduften und so das Wohlbefinden einer in einem Raum befindlichen Person zu steigern. Außerdem werden dadurch schlechte Raumgerüche vermieden.

Die Fördereinrichtung dient neben dem Ein- und/oder Ausbringen von Luft in die und/oder aus der Vorrichtung dazu, die Luft in dem Raum umzuwälzen.

Durch das Filtern der Luft können durch Bakterien- und Virenbefall verursachte Krankheiten sowie allergische Reaktionen und gesundheitliche Probleme aufgrund von Pollen vermieden werden. Außerdem wird auch die Feinstaubbelastung gesenkt und Umweltgifte aus der Luft gefiltert, was wiederum das Risiko für Gesundheitsschäden senkt.

Insbesondere wenn Zirbenholz zum Beduften des Raumes zum Einsatz kommt, hat dies weitere positive Effekte auf eine in dem Raum befindliche Person. So kann der Herzschlag der im Raum befindlichen Person um bis zu 3500 Schläge pro 24 Stunden reduziert werden, was der Lebensdauer der im Raum befindlichen Person zugutekommt. Außerdem können die Konzentrationsfähigkeit sowie die Schlafqualität der im Raum befindlichen Person gesteigert werden.

Wenn eine Bepflanzungseinrichtung, welche zumindest eine lebende Pflanze, vorzugsweise ein Moos, eine Flechte und/oder eine Blütenpflanze, umfasst vorgesehen ist, so kann die lebende Pflanze der Bepflanzungseinrichung einen Teil des in der Luft befindlichen CO₂ in Sauerstoff umwandeln. Somit wird die Luft mit natürlichem Sauerstoff angereichert. Die lebende Pflanze trägt außerdem zu einer angenehmen Atmosphäre in dem Raum bei.

Weitere vorteilhafte Ausführungsformen der Erfindung werden in den abhängigen Ansprüchen definiert.

Bevorzugt kann die Vorrichtung eine Desinfektionseinrichtung zum Desinfizieren der eingebrachten Luft umfassen. Dadurch können Bakterien und Viren, welche durch die Filtereinrichtung nicht herausgefiltert wurden, abgetötet werden. Es wird also sichergestellt, dass die Belastung der Luft durch Bakterien und Viren kleinstmöglich ist.

Bevorzugt kann auch vorgesehen sein, dass die Fördereinrichtung in Form eines, vorzugsweise drehzahlgeregelten, Ventilators ausgebildet ist. Das stellt eine einfache und effektive Möglichkeit dar, eine Fördereinrichtung umzusetzen. Ist der Ventilator drehzahlgeregelt, so kann eine durch die Förderungseinrichtung geförderte Luftmenge an die Größe und/oder eine vorhandene Raumluftqualität des Raums angepasst werden.

Insbesondere kann vorgesehen sein, dass die Filtereinrichtung zumindest zwei Filter aufweist. Es kann auch vorgesehen sein, dass die Filtereinrichtung zumindest einen, vorzugsweise mehrere der folgenden Filter aufweist:
- Vorfilter
- Schwebstofffilter, insbesondere als HEPA-Filter,
- Aktivkohlefilter
- Trockenfilter, und/oder
- Zyklonfilter.

Dadurch kann auf einfache Weise die Zahl und die Art der Filter der Filtereinrichtung auf gegebene Umstände, wie Grad der Feinstaub oder Schadstoffbelastung, angepasst werden.

Besonders bevorzugt kann vorgesehen sein, dass die Beduftungseinrichtung zumindest einen Beduftungsbehälter aufweist, welcher vorzugsweise mit Holz, insbesondere mit Zirbenholz gefüllt ist, wobei der zumindest eine Beduftungsbehälter von der eingebrachten Luft durch- und/oder überströmbar ist. Die Zahl der Beduftungsbehälter hängt dabei von der Größe des Raumes sowie der Größe der erfindungsgemäßen Vorrichtung ab.

Es kann auch vorgesehen sein, dass die Beduftungseinrichtung eine Beduftungsanlage zur Beduftung der eingebrachten Luft über ätherische Öle, Duftstoff-Essenzen und/oder ätherische Essenzen aufweist. Die ätherischen Öle, Duftstoff-Essenzen und/oder ätherische Essenzen werden in die eingebrachte Luft eingeimpft oder verdunstet, wodurch die eingebrachte Luft nicht nur beduftet, sondern auch gereinigt und zusätzlich desinfiziert wird.

Bevorzugt kann vorgesehen sein, dass die Bepflanzungseinrichtung einen Pflanzenträger eine Befeuchtungseinheit für den Pflanzenträger und/oder eine Sensoreinheit aufweist.

Der Pflanzenträger dient dabei zum Tragen der lebenden Pflanze und auch als Nährstoffspeicher. Dazu kann vorgesehen sein, dass der Pflanzenträger eine Gitterstruktur aufweist, wobei die Gitterstruktur vorzugsweise mit nährstoffhaltigem Material gefüllt ist.

Die Befeuchtungseinheit stellt die Wasserversorgung der lebenden Pflanze sicher und kann dazu ein Wasserreservoir, eine Pumpe, eine Anschlusseinheit und/oder ein Verteilerelement umfassen. Über das Verteilerelement wird Wasser an die lebende Pflanze abgegeben. Das Wasser kann einerseits aus dem Wasserreservoir über eine Pumpe zum Verteilerelement gefördert werden oder über das Anschlusselement einer Wasserleitung entnommen werden. Das Wasser dient weiters dazu, die lebende Pflanze mit Nährstoffen zu versorgen. Diese Nährstoffe können auch in flüssiger Form über die Wasserzuführung an die Pflanze transportiert werden.

Die Sensoreinheit dient zur Überwachung verschiedener Kennwerte der lebenden Pflanze bzw. des Pflanzenträgers. Dazu kann vorgesehen sein, dass die Sensoreinheit ein Wachstum, einen Nährstoffgehalt und/oder einen Feuchtigkeitsgehalt am oder im Pflanzenträger erfasst.

Besonders bevorzugt kann vorgesehen sein, dass die Pumpe und/oder das Verteilerelement in Abhängigkeit des erfassten Wachstums und/oder des Feuchtigkeitsgehalts am Pflanzenträgers gesteuert und/oder geregelt werden. Dadurch wird sichergestellt, dass die lebende Pflanze stets ausreichend mit Wasser versorgt wird und somit optimal gedeihen kann. Auch die Zuführung der Nährstoffe kann damit gesteuert oder geregelt werden.

Es kann vorgesehen sein, dass die Desinfektionseinrichtung zumindest eine UV-Lichtquelle, vorzugsweise eine UV-C-Lichtquelle, aufweist. Bevorzugt kann vorgesehen sein, dass eine Vielzahl an UV-Lichtquellen, vorzugsweise eine Vielzahl an UV-C-Lichtquellen, vorgesehen sind. Das stellt eine unkomplizierte Möglichkeit zur Realisierung der Desinfektionseinrichtung dar.

Wenn die Desinfektionseinrichtung zumindest einen Spiegel vorzugsweise mehrere Spiegel, zur Reflexion eines durch die zumindest eine UV-Lichtquelle emittierten UV-Lichts aufweist, so wird die desinfizierende Wirkung des emittierten UV-Lichts durch die Reflexion desselben verstärkt.

In einer Ausführungsform der Erfindung kann die Vorrichtung eine Befeuchtungseinrichtung zum Befeuchten der eingebrachten Luft und/oder des Holzes umfassen. Somit kann die Luftfeuchtigkeit, vor allem während einer Heizperiode, auf ein ideales Maß angehoben werden. Wird das Holz befeuchtet, so können Duftstoffe des Holzes effektiver in die eingebrachte Luft eingebracht werden.

Besonders bevorzugt kann vorgesehen sein, dass die Vorrichtung eine Sensoreinrichtung aufweist, welche zumindest eine der folgenden Daten erfasst:
- Luftfeuchtigkeit,
- Lufttemperatur,
- Luftdruck,
- CO2-Gehalt,
- CO-Gehalt,
- Sauerstoffgehalt der Luft,
- Flüchtige organische Verbindungen (VOC),
- Feinstaubbelastung,
- Verschmutzung von Filtern,
- Zustand einer Beduftungseinrichtung, und/oder
- Zustand der Viren- und Bakterien-Belastung in der Luft.

Somit können objektive Kriterien der Raumluftqualität überprüft werden.

Wenn die Vorrichtung eine Steuer-/Regeleinheit aufweist und wenn die Steuer-Regeleinheit zumindest eine der Komponenten der Vorrichtung, vorzugsweise in Abhängigkeit von durch die Sensoreinheit und/oder die Sensoreinrichtung bereitgestellten Daten, steuert und/oder regelt:
- Fördereinrichtung,
- Beduftungseinrichtung,
- Befeuchtungseinheit, insbesondere Pumpe und/oder Verteilerelement,
- Desinfektionseinrichtung,
- Befeuchtungseinrichtung, und/oder
- Nährstoffzuführung zum Pflanzenträger,
dann ist es möglich, die Raumluftqualität automatisiert zu regeln und dadurch stets eine ideale Raumluftqualität sicherzustellen.

Die Vorrichtung kann ein Gehäuse zur Aufnahme der Fördereinrichtung, der Filtereinrichtung und/oder der Beduftungsvorrichtung aufweisen.

Besonders bevorzugt kann vorgesehen sein, dass die Bepflanzungseinrichtung zumindest teilweise außen am Gehäuse, vorzugsweise an einer Tür des Gehäuses, angeordnet ist. Dadurch kann die lebende Pflanze direkt mit der Luft in einem Raum interagieren. Außerdem dient die lebende Pflanze dadurch auch als Zierelement und trägt damit zum Wohlbefinden einer in dem Raum befindlichen Person bei.

Es kann auch vorgesehen sein, dass das Gehäuse im Wesentlichen quaderförmig oder zylinderförmig ausgebildet ist.

Auch vorgesehen sein kann, dass das Gehäuse Befestigungsmittel zur Befestigung der Vorrichtung einer vorzugsweise vertikalen Wand aufweist.

Weiters kann vorgesehen sein, dass das Gehäuse Trägermittel, insbesondere Sockel oder Standfüße, zum Aufstellen der Vorrichtung auf einem Boden aufweist.

Wenn das Gehäuse demgemäß ausgebildet ist, so ist eine einfache und platzsparende Montage der erfindungsgemäßen Vorrichtung in einem Raum möglich. Die erfindungsgemäße Vorrichtung lässt sich als einfach in jeden beliebigen Raum nachrüsten.

Bevorzugt kann vorgesehen sein, dass die Vorrichtung eine Anzeigevorrichtung aufweist, welche zumindest eine der folgenden Daten anzeigt:
- Luftqualität,
- Luftfeuchtigkeit,
- Lufttemperatur,
- Luftdruck,
- CO2-Gehalt,
- CO-Gehalt,
- Flüchtige organische Verbindungen (VOC),
- Feinstaubbelastung,
- Verschmutzung von Filtern,
- Zustand einer Beduftungseinrichtung,
- Füllstand eines Wasserreservoirs,
- Feuchtigkeitsgehalt eines Pflanzenträgers,
- Menge/Zustand von Nährstoffen an oder in einem Pflanzenträger,
- Uhrzeit,
- Luftwechsel, und/oder
- Viren- und Bakterien-Belastung.

Dadurch ist es einem Benutzer der erfindungsgemäßen Vorrichtung einerseits möglich die Raumluftqualität zu überprüfen und andererseits den Zustand und die Funktion der erfindungsgemäßen Vorrichtung zu überprüfen.

Wenn die Anzeigevorrichtung eine Eingabeeinheit, vorzugsweise in Form eines Touch-Displays, umfasst und wenn der Steuer-/Regeleinheit über die Eingabeeinheit Daten, Einstellungen und/oder Parameter übergeben werden können, ist es auf einfache Weise möglich, die erfindungsgemäße Vorrichtung einzustellen, zu kalibrieren, usw.

Weitere Einzelheiten und Vorteile der Erfindung werden anhand der Figurenbeschreibung unter Bezugnahme auf die Zeichnungen im Folgenden näher erläutert. Darin zeigen:
- Fig. 1: eine schematische Darstellung einer erfindungsgemäßen Vorrichtung in einer geöffneten Stellung,
- Fig. 2a: eine schematische Darstellung einer erfindungsgemäßen Vorrichtung in einer geschlossenen Stellung,
- Fig. 2b: eine schematische Darstellung einer erfindungsgemäßen Vorrichtung unter Ausblendung einer lebenden Pflanze,
- Fig. 3a: eine schematische Schnittdarstellung einer erfindungsgemäßen Vorrichtung in einer geschlossenen Stellung, und
- Fig. 3b: eine schematische Schnittdarstellung einer erfindungsgemäßen Vorrichtung in einer geöffneten Stellung, und
- Fig. 3c: eine weitere schematische Schnittdarstellung einer erfindungsgemäßen Vorrichtung in einer geschlossenen Stellung.

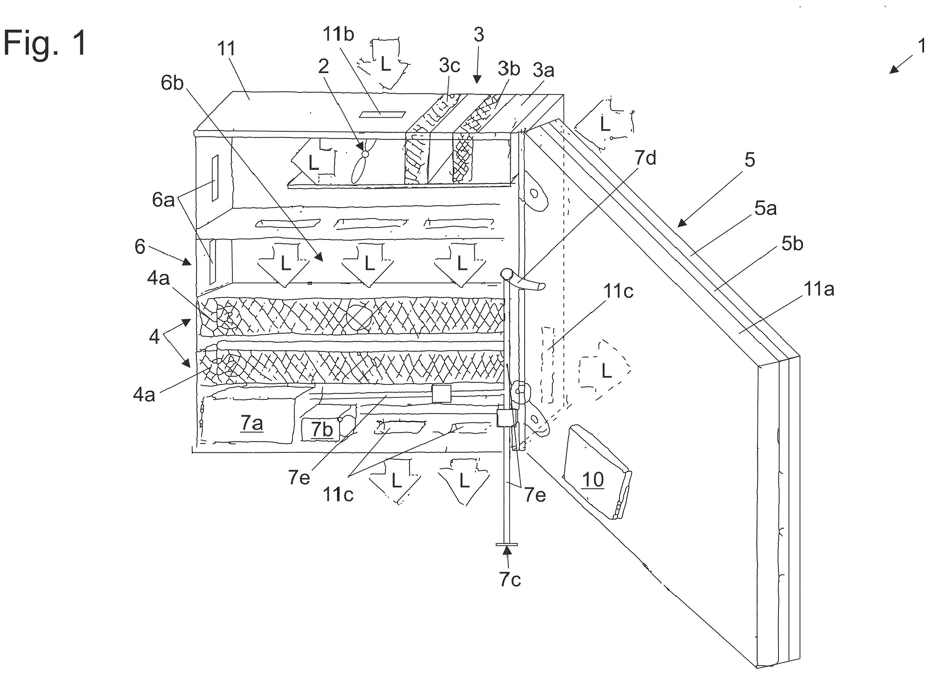

Die Figur 1 zeigt eine schematische Darstellung einer erfindungsgemäßen Vorrichtung 1 in einer geöffneten Stellung. Es ist ein quaderförmiges Gehäuse 11 mit einer Tür 11a erkennbar. Am Gehäuse 11 sind Einlassöffnungen 11b und Auslassöffnungen 11c zum Ein- bzw. Ausbringen von Luft L in die Vorrichtung 1 angeordnet.

Nach dem Eintreten der Luft L in die Vorrichtung 1, wird die Luft L über Filter 3a, 3b und 3c der Filtereinrichtung 3 gefiltert. Der Filter 3a ist dabei als grober Filter, der Filter 3b als Aktivkohle-Filter und der Filter 3c als HEPA-Filter ausgebildet. Somit ist eine optimale Filterung der Luft L möglich.

Es ist auch denkbar, eine andere Anzahl an Filtern oder andere Arten von Filtern einzusetzen.

Die Luft L wird über eine Fördereinrichtung 2 in Form eines drehzahlgeregelten Ventilators in Abhängigkeit der Größe eines Raums oder der vorhandenen Raumluftqualität des Raums gefördert.

Nach dem Filtern der Luft L wird die Luft L in einer Desinfektionseinrichtung 6 durch aus UV-Lichtquellen 6a emittiertes UV-C Licht desinfiziert. Es ist erkennbar, dass bereits vor der Desinfektionseinrichtung 6 UV-Lichtquellen 6a angeordnet sein können.

Die Desinfektionseinrichtung 6 umfasst weiters Spiegel 6b, welche das emittierte UV-C Licht reflektieren und so die Desinfektionsleistung der Desinfektionseinrichtung 6 erhöhen.

Der Desinfektionseinrichtung 6 nachgeschaltet ist eine Beduftungseinrichtung 4. Die Beduftungseinrichtung 4 umfasst im vorliegenden Ausführungsbeispiel zwei Beduftungsbehälter 4a und eine nicht dargestellte Beduftungsanlage 4b. Die Zahl der Beduftungsbehälter 4a kann in Abhängigkeit der Größe eines Raums oder der Größe der Vorrichtung 1 variiert werden.

Die Beduftungsbehälter 4a beinhalten im vorliegenden Ausführungsbeispiel Zirbenholz. Über- oder durchströmt die Luft L die Beduftungsbehälter 4a, so nimmt die Luft Duftstoffe des Zirbenholzes auf.

Das Zirbenholz kann beispielsweise als Locken oder als Gitterstruktur vorliegen. Wichtig dabei ist, dass die Oberfläche des Zirbenholzes maximiert wird.

Es kann auch eine andere Holzart oder mehrere Holzarten in den Beduftungsbehältern 4a vorgesehen sein.

Nach dem Beduften der Luft L tritt die Luft L über Austrittsöffnungen 11c aus der Vorrichtung 1 aus und wieder in einen Raum ein.

Im vorliegenden Ausführungsbeispiel ist weiters eine nicht dargestellte Befeuchtungseinrichtung 8 zur Befeuchtung der Luft L vorgesehen. Alternativ oder zusätzlich dazu kann vorgesehen sein, dass die Befeuchtungseinrichtung 8 das Holz in den Beduftungsbehältern befeuchtet.

An der Tür 11a ist ein Pflanzenträger 5b, an welchem die lebende Pflanze, im vorliegenden Ausführungsbeispiel ein Moos, angeordnet ist, angeordnet. Es können auch andere Pflanzenarten vorgesehen sein.

Über einen nicht dargestelltes Verteilerelement 7d kann die lebende Pflanze 5a bzw. der Pflanzenträger 5b mit Wasser versorgt werden. Das Wasser wird dabei entweder aus einem Wasserreservoir 7a über eine Pumpe 7b über eine Leitung 7e zum Verteilerelement 7d gefördert oder über eine Anschlussvorrichtung 7c aus einer Wasserleitung entnommen werden. Die Leitung 7e weist dabei einen Verbindungsabschnitt 7f auf. Der Verbindungsabschnitt 7f kann beispielsweise als Schlauch ausgebildet und dient dazu, eine Verbindung zwischen der Leitung 7e im Gehäuse 11 und dem Verteilerelement 7d an der Tür 11a herzustellen.

Im vorliegenden Ausführungsbeispiel ist eine Steuer-/Regeleinheit 10 an der Tür 11a angeordnet. Die Steuer-/Regeleinheit 10 kann aber grundsätzlich an jeder geeigneten Position angeordnet werden.

Es ist weiters eine Sensoreinrichtung 9 umfassend eine Vielzahl an verschiedenen Sensoren vorgesehen, welche allerdings nicht dargestellt ist.

Die Steuer-/Regeleinheit 10 kann in Abhängigkeit von von der Sensoreinrichtung 9 und einer Sensoreinheit 5c gemessenen Daten zumindest eine der Komponenten der Vorrichtung 1 steuern und/oder regeln.

Weiters ist eine nicht dargestellte Beduftungsanlage 4b zur Beduftung der eingebrachten Luft über ätherische Öle, Duftstoff-Essenzen und/oder ätherische Essenzen vorgesehen.

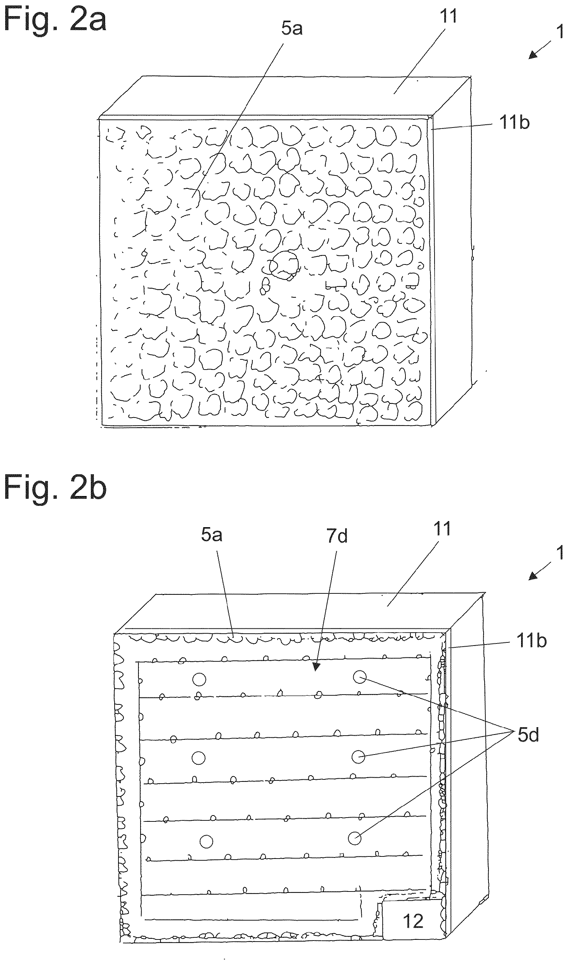

Die Figur 2a zeigt eine schematische Darstellung einer erfindungsgemäßen Vorrichtung 1 in einer geschlossenen Stellung. Es ist erkennbar, dass an der Tür 11b eine lebend Pflanze 5a in Form eines Mooses angeordnet ist. Insgesamt ist das Gehäuse annähernd plattenförmig ausgebildet.

Die Figur 2b zeigt eine schematische Darstellung einer erfindungsgemäßen Vorrichtung 1 unter weitgehender Ausblendung einer lebenden Pflanze 5a. Es ist erkennbar, dass unterhalb der lebenden Pflanze 5a ein Pflanzenträger 5b angeordnet ist, auf welchem die lebenden Pflanze 5a angeordnet ist. Außerdem ist ein Verteilerelement 7d zur Bewässerung der lebenden Pflanze 5a ersichtlich.

Weiters sind Sensoren 5d einer nicht dargestellten Sensoreinheit 5c erkennbar, welche den Feuchtigkeitsgehalt am Pflanzenträger 5b bzw. der lebenden Pflanze 5a misst. Die Sensoren 5d können auch dazu ausgebildet sein, das Wachstum der lebenden Pflanze 5a zu messen.

In Abhängigkeit des gemessenen Feuchtigkeitsgehalts und/oder des Wachstums kann die lebende Pflanze 5a bewässert werden. Die Bewässerung ist weiters auch von der Art der Pflanze abhängig. Insbesondere werden in Abhängigkeit des gemessenen Feuchtigkeitsgehalts und/oder des Wachstums die Pumpe 7b und oder die Wasserzufuhr über die Anschlussvorrichtung 7c gesteuert bzw. geregelt.

Im Ausführungsbeispiel gemäß Figur 2b ist eine Anzeigevorrichtung 12 in Form eines Touchscreens an der Tür 11a angeordnet. Es ist auch denkbar, dass eine Eingabeeinheit der Anzeigevorrichtung in Form von Knöpfen, Drehreglern usw. ausgebildet ist.

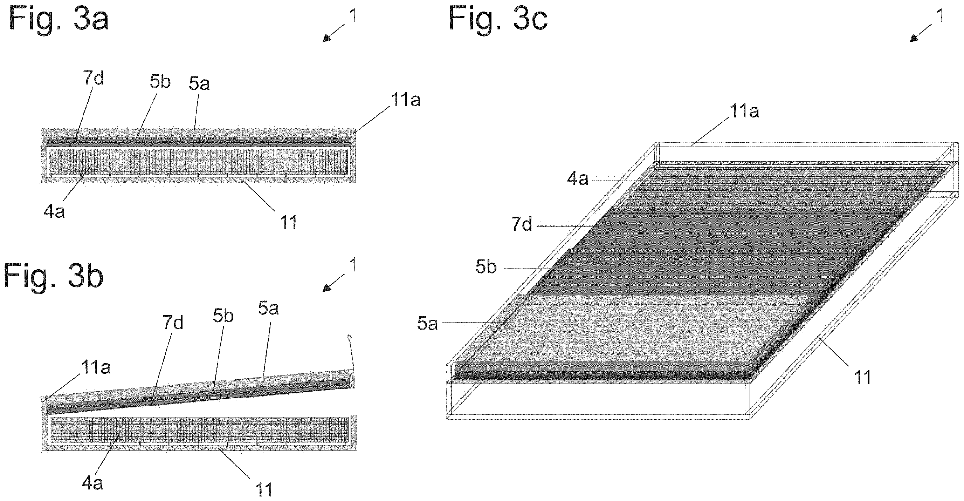

Die Figur 3a zeigt eine schematische Schnittdarstellung einer erfindungsgemäßen Vorrichtung 1 in einer geschlossenen Stellung und die Figur 3b eine schematische Schnittdarstellung einer erfindungsgemäßen Vorrichtung 1 in einer geöffneten Stellung und die Figur 3c eine weitere schematische Schnittdarstellung einer erfindungsgemäßen Vorrichtung 1 in einer geschlossenen Stellung.

Es ist erkennbar, dass das Verteilerelement 7d unterhalb des Pflanzenträgers 5b angeordnet ist. Die lebende Pflanze 5a ist am Pflanzenträger 5b angeordnet bzw. mit dem Pflanzenträger 5b verwachsen. Es ist jeweils auch ein Beduftungsbehälter 4a innerhalb des Gehäuses 11 erkennbar.

### Bezugszeichenliste:

- 1: Vorrichtung
- 2: Fördereinrichtung
- 3: Filtereinrichtung
- 3a: grober Vorfilter
- 3b: Aktivkohle-Filter
- 3c: HEPA-Filter
- 4: Beduftungseinrichtung
- 4a: Beduftungsbehälter
- 4b: Beduftungsanlage
- 5: Bepflanzungseinrichtung
- 5a: lebende Pflanze
- 5b: Pflanzenträger
- 5c: Sensoreinheit
- 5d: Feuchtesensor
- 6: Desinfektionseinrichtung
- 6a: UV-Lichtquelle
- 6b: Spiegel
- 7: Befeuchtungseinheit
- 7a: Wasserreservoir
- 7b: Pumpe
- 7c: Anschlusseinheit
- 7d: Verteilerelement
- 7e: Leitung
- 7f: Verbindungselement
- 8: Befeuchtungseinrichtung
- 9: Sensoreinrichtung
- 10: Steuer-/Regeleinheit
- 11: Gehäuse
- 11a: Tür
- 11b: Eintrittsöffnung
- 11c: Austrittsöffnung
- 11d: Befestigungsmittel
- 11e: Trägermittel
- 12: Anzeigevorrichtung

## Patentansprüche

1. Vorrichtung (1) zur Verbesserung einer Raumluftqualität umfassend:
- eine Fördereinrichtung (2), insbesondere einen drehzahlgeregelten Ventilator, zum Ein- und/oder Ausbringen von Luft in die und/oder aus der Vorrichtung (1),
- eine Filtereinrichtung (3) zum Filtern der in die Vorrichtung eingebrachten Luft, und
- eine Beduftungseinrichtung (4) zur Beduftung der eingebrachten Luft durch Holz, insbesondere durch Zirbenholz, und/oder ätherische Essenzen,
- vorzugweise eine von der Beduftungseinrichtung (4) gesonderte Bepflanzungseinrichtung (5) aufweist, welche zumindest eine lebende Pflanze (5a), vorzugsweise ein Moos, eine Flechte und/oder eine Blütenpflanze, umfasst.

2. Vorrichtung nach Anspruch 1, **dadurch gekennzeichnet, dass** die Vorrichtung (1) eine Desinfektionseinrichtung (6) zum Desinfizieren der eingebrachten Luft umfasst.

3. Vorrichtung nach einem der Ansprüche 1 oder 2, **dadurch gekennzeichnet, dass** die Filtereinrichtung (3) zumindest einen, vorzugsweise mehrere der folgenden Filter aufweist:
- Vorfilter
- Schwebstofffilter, insbesondere als HEPA-Filter,
- Aktivkohlefilter
- Trockenfilter, und/oder
- Zyklonfilter.

4. Vorrichtung nach einem der Ansprüche 1 bis 3, **dadurch gekennzeichnet, dass** die Beduftungseinrichtung (4) zumindest einen Beduftungsbehälter (4a) aufweist, welcher vorzugsweise mit Holz, insbesondere mit Zirbenholz gefüllt ist, wobei der zumindest eine Beduftungsbehälter (4a) von der eingebrachten Luft durch- und/oder überströmbar ist, wobei vorzugsweise vorgesehen ist, dass die Beduftungseinrichtung (4) eine Beduftungsanlage (4b) zur Beduftung der eingebrachten Luft über ätherische Öle, Duftstoff-Essenzen und/oder ätherische Essenzen aufweist.

5. Vorrichtung nach einem der Ansprüche 1 bis 4, **dadurch gekennzeichnet, dass** die Bepflanzungseinrichtung (5) einen vorzugsweise eine Gitterstruktur aufweisenden Pflanzenträger (5b) und eine Befeuchtungseinheit (7) oder eine gegebenenfalls auch Nährstoffe für die Pflanzen zuführende Bewässerungseinheit für den Pflanzenträger (5b) oder die Pflanzen und vorzugsweise eine Sensoreinheit (5c) zum Erfassen der Feuchte aufweist.

6. Vorrichtung nach einem der Ansprüche 2 bis 5, **dadurch gekennzeichnet, dass** die Desinfektionseinrichtung (6) zumindest eine UV-Lichtquelle (6a), vorzugsweise eine UV-C-Lichtquelle, aufweist.

7. Vorrichtung nach Anspruch 6, **dadurch gekennzeichnet, dass** die Desinfektionseinrichtung (6) zumindest einen Spiegel (6b) vorzugsweise mehrere Spiegel (6b), zur Reflexion eines durch die zumindest eine UV-Lichtquelle (6a) emittierten UV-Lichts aufweist.

8. Vorrichtung nach einem der Ansprüche 1 bis 7, **dadurch gekennzeichnet, dass** die Vorrichtung (1) eine Befeuchtungseinrichtung (8) zum Befeuchten der eingebrachten Luft und/oder des Holzes umfasst.

9. Vorrichtung nach einem der Ansprüche 1 bis 8, **dadurch gekennzeichnet, dass** die Vorrichtung (1) eine Sensoreinrichtung (9) aufweist, welche zumindest eine, vorzugsweise mehrere, der folgenden Daten erfasst:
- Luftfeuchtigkeit,
- Lufttemperatur,
- Luftdruck,
- CO₂-Gehalt,
- CO-Gehalt,
- Sauerstoffgehalt der Luft,
- Flüchtige organische Verbindungen (VOC),
- Feinstaubbelastung,
- Verschmutzung von Filtern,
- Zustand einer Beduftungseinrichtung, und/oder
- Zustand der Viren- und Bakterien-Belastung in der Luft.

10. Vorrichtung nach einem der Ansprüche 1 bis 9, **dadurch gekennzeichnet, dass** die Vorrichtung (1) eine Steuer-/Regeleinheit (10) aufweist, wobei vorzugsweise vorgesehen ist, dass die Sensoreinrichtung (9) einen CO₂-Gehalt erfasst, wobei die Steuer-/Regeleinheit (10) in Abhängigkeit des erfassten CO₂-Gehalts zumindest eine Komponente der Vorrichtung (1), vorzugsweise die Fördereinrichtung (2), steuert und/oder regelt.

11. Vorrichtung nach Anspruch 10, **dadurch gekennzeichnet, dass** die Steuer-Regeleinheit (10) zumindest eine der Komponenten der Vorrichtung (1), vorzugsweise in Abhängigkeit von durch die Sensoreinheit (5c) und/oder die Sensoreinrichtung (9) bereitgestellten Daten, steuert und/oder regelt:
- Fördereinrichtung (2),
- Beduftungseinrichtung (4),
- Befeuchtungseinheit (7), insbesondere Pumpe (7b) und/oder Verteilerelement (7d),
- Desinfektionseinrichtung (6),
- Befeuchtungseinrichtung (8), und/oder
- Nährstoffzuführung zum Pflanzenträger.

12. Vorrichtung nach einem der Ansprüche 1 bis 11, **dadurch gekennzeichnet, dass** die Vorrichtung (1) ein Gehäuse (11) zur Aufnahme der Fördereinrichtung (2), der Filtereinrichtung (3) und/oder der Beduftungsvorrichtung (4) aufweist.

13. Vorrichtung nach Anspruch 12, **dadurch gekennzeichnet, dass** die Bepflanzungseinrichtung (5) zumindest teilweise außen am Gehäuse (11), vorzugsweise an einer Tür (11a) des Gehäuses (11), angeordnet ist.

14. Vorrichtung nach einem der Ansprüche 12 oder 13 und 4, **dadurch gekennzeichnet, dass** der zumindest eine Beduftungsbehälter (4a) lösbar im Gehäuse (11) angeordnet oder anordenbar ist.

15. Vorrichtung nach einem der Ansprüche 1 bis 14, **dadurch gekennzeichnet, dass** die Vorrichtung (1) eine Anzeigevorrichtung (12) aufweist, welche zumindest eine der folgenden Daten anzeigt:
- Luftqualität,
- Luftfeuchtigkeit,
- Lufttemperatur,
- Luftdruck,
- CO₂-Gehalt,
- CO-Gehalt,
- Flüchtige organische Verbindungen (VOC),
- Feinstaubbelastung,
- Verschmutzung von Filtern,
- Zustand einer Beduftungseinrichtung,
- Füllstand eines Wasserreservoirs,
- Feuchtigkeitsgehalt eines Pflanzenträgers,
- Menge/Zustand von Nährstoffen an oder in einem Pflanzenträger,
- Uhrzeit, und/oder
- Luftwechsel,
wobei vorzugsweise vorgesehen ist, dass die Anzeigevorrichtung (12) eine Eingabeeinheit, vorzugsweise in Form eines Touch-Displays, umfasst.
